# EUROPEAN PATENT APPLICATION

(11) **EP 4 296 267 A1**
(43) Date of publication of application: **27.12.2023**
(21) Application number: 22756333.5
(22) Date of filing: 22.02.2022
(51) Int. Cl.: C07D 249/08

(54) **METHOD FOR MANUFACTURING AZOLE DERIVATIVE**

(30) Priority: 22.02.2021 JP 2021026670
(71) Applicant: Kureha Corporation, Chuo-ku Tokyo 103-8552 (JP)
(72) Inventor: NAGAI, Kaito, Tokyo 103-8552 (JP); HARIGAE, Ryo, Tokyo 103-8552 (JP)
(74) Representative: Zimmermann & Partner Patentanwälte mbB
(86) International application number: PCT/JP2022/007064
(87) International publication number: WO 2022/177016

(57) **Abstract**

Provided is a method for producing an azole derivative with less amount of a by-product. A method for producing an azole derivative represented by general formula (I) includes reacting an oxirane derivative with 4-amino-1,2,4-triazole under an acidic condition and deaminating the obtained compound to form the azole derivative represented by general formula (I), where R¹ is a C₁-C₆-alkyl group or the like; X¹ and X² are each independently a halogen group or the like; and n is 1, 2, or 3.

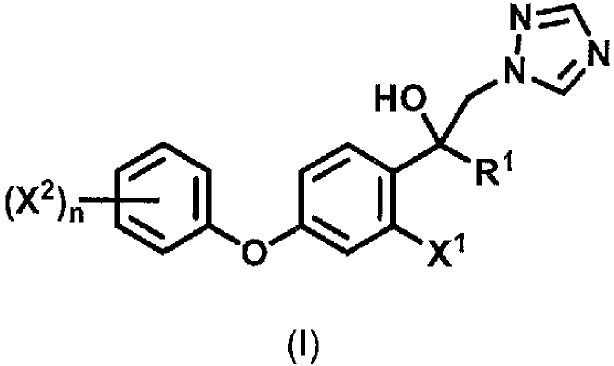

## Description

### TECHNICAL FIELD

The present invention relates to a method for producing an azole derivative.

### BACKGROUND ART

An azole derivative described in Patent Document 1 is known as an agricultural or horticultural chemical that exhibits a high controlling effect.

Patent Document 1 also describes a method for producing the azole derivative, in which an oxirane compound is reacted with sodium azole to form an azole.

### Citation List

### Patent Literature

Patent Document 1: WO 2019/093522 A1

### SUMMARY OF INVENTION

### TECHNICAL PROBLEM

As a result of studies, the inventors of the present invention have found that when the azole is a triazole, a 1 ,3,4-triazole form is produced as a by-product in addition to a target 1,2,4-triazole form in the formation of an azole. When the by-product is formed, there arises a problem that the yield and purity of the target compound decreases. Further, there arises a problem that the burden of post-treatment for removing the by-product increases.

The present invention has been made in view of the above problems, and an object of the present invention is to provide a method for producing an azole derivative with which the amount of the by-product is reduced as compared with existing production methods.

### SOLUTION TO PROBLEM

To solve the above problem, a method for producing an azole derivative according to the present invention is a method for producing an azole derivative represented by general formula (I): 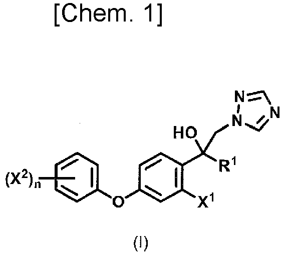 where in general formula (I),
R¹ is a C₁-C₆-alkyl group or CO₂R², where R² is a C₁-C₆-alkyl group;
X¹ and X² are each independently a halogen group, a C₁-C₄-haloalkyl group, or a C₁-C₄-haloalkoxy group;
and n is 1, 2, or 3,
the method including:
   reacting an oxirane derivative represented by general formula (II) with 4-amino-1,2,4-triazole under an acidic condition to obtain a compound represented by general formula (Ia), 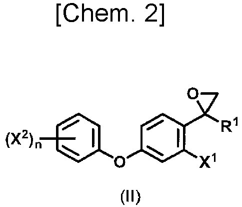 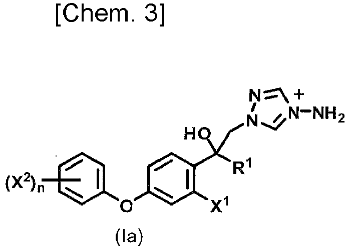
   where in general formulae (II) and (Ia), R¹, X¹, X², and n are respectively the same as R¹, X¹, X², and n in general formula (I); and deaminating the obtained compound represented by general formula (Ia) to form an azole derivative represented by general formula (I).

### ADVANTAGEOUS EFFECTS OF INVENTION

According to the present invention, the target azole derivative can be produced with less amount of a by-product.

### DESCRIPTION OF EMBODIMENTS

A preferred embodiment for carrying out the present invention will be described below.

The present embodiment is a method for producing an azole derivative represented by general formula (I) (hereinafter referred to as "azole derivative (I)").

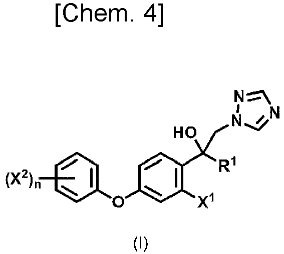

In formula (I), R¹ represents a C₁-C₆-alkyl group or CO₂R². R² represents a C₁-C₆-alkyl group.

The C₁-C₆-alkyl group is a linear or branched alkyl group having 1 to 6 carbon atoms. Specific examples of the C₁-C₆-alkyl group include a methyl group, an ethyl group, a 1-methylethyl group, a 1,1-dimethylethyl group, a propyl group, a 1-methylpropyl group, a 2-methylpropyl group, a 1,1-dimethylpropyl group, a 2,2-dimethylpropyl group, a 1-ethylpropyl group, a butyl group, a 1-methylbutyl group, a 2-methylbutyl group, a 3-methylbutyl group, a 3,3-dimethylbutyl group, a 2,2-dimethylbutyl group, a 1,1-dimethylbutyl group, a 1-ethylbutyl group, a 2-ethylbutyl group, a pentyl group, a 1-methylpentyl group, a 2-methylpentyl group, a 3-methylpentyl group, and a 4-methylpentyl group.

X¹ and X² are each independently a halogen group, a C₁-C₄-haloalkyl group, or a C₁-C₄-haloalkoxy group.

Examples of the halogen group include a chlorine group, a bromine group, an iodine group, and a fluorine group.

The C₁-C₄-haloalkyl group has one or more halogen atoms as substituents at substitutable positions of the C₁-C₄-alkyl group, and in a case of substitution with two or more halogen groups, the halogen groups may be the same or different. The C₁-C₄-alkyl group is a linear or branched alkyl group having 1 to 4 carbon atoms.

The C₁-C₄-alkyl group is a linear or branched alkyl group having 1 to 4 carbon atoms, and examples thereof include a methyl group, an ethyl group, a propyl group, and a butyl group. The halogen group is as described above. Examples of the C₁-C₄-haloalkyl group include a chloromethyl group, a 2-chloroethyl group, a 2,3-dichloropropyl group, a bromomethyl group, a chlorodifluoromethyl group, a trifluoromethyl group, and a 3,3,3-trifluoropropyl group.

The C₁-C₄-haloalkoxy group has one or more halogen atoms as substituents at substitutable positions of the C₁-C₄-alkoxy group, and in a case of substitution with two or more halogen groups, the halogen groups may be the same or different. The C₁-C₄-alkoxy group is a linear or branched alkoxy group having 1 to 4 carbon atoms.

The C₁-C₄-alkoxy group is a linear or branched alkoxy group having 1 to 4 carbon atoms, and examples thereof include a methoxy group, an ethoxy group, a propoxy group, an isopropoxy group, a 1-methylpropoxy group, a 2-methylpropoxy group, a butoxy group, and a 1,1-dimethylethoxy group. Examples of the C₁-C₄-haloalkoxy group include a trifluoromethoxy group, a difluoromethoxy group, a 1,1,2,2,2-pentafluoroethoxy group, and a 2,2,2-trifluoroethoxy group.
n is 1, 2, or 3. When n is 2 or 3, a plurality of X² may be the same or different.

The production method according to the present embodiment includes a step (hereinafter referred to as "Step 1") of reacting an oxirane derivative represented by general formula (II) (hereinafter referred to as "oxirane derivative (II)") with 4-amino-1,2,4-triazole to obtain an amine compound represented by general formula (Ia) (hereinafter referred to as "amine compound (Ia)") and a step (hereinafter referred to as "Step 2") of deaminating the amine compound (Ia) to form an azole derivative (I) according to the following reaction scheme. R¹, X¹, X², and n in the reaction scheme correspond to R¹, X¹, X², and n in general formula (I) described above.

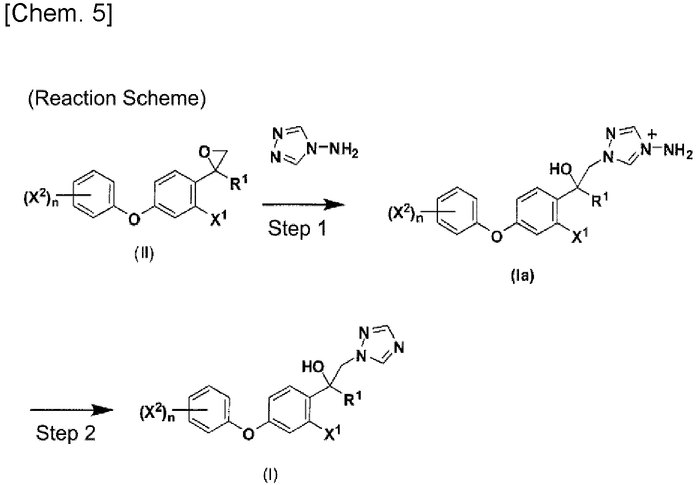

### Step 1

In Step 1, the oxirane derivative (II) is reacted with 4-amino-1,2,4-triazole under an acidic condition to obtain the amine compound (Ia).

An amount of 4-amino-1,2,4-triazole added to the reaction system in Step 1 is preferably 1.0 to 3.0 equivalent (eq.) with respect to 1 equivalent (eq.) of the oxirane derivative (II) from the perspective of adequately performing the reaction of Step 1.

When the reaction is performed under an acidic condition, an acid used for forming the acidic condition is not particularly limited as long as it is an acid capable of maintaining the acidic condition during the reaction, but the acid is preferably an acid having a pKₐ of 0 or less. Examples of the acid having a pKₐ of 0 or less include an alkylsulfonic acid, an arylsulfonic acid, a haloalkylsulfonic acid, and sulfuric acid. Of these, an alkylsulfonic acid and an arylsulfonic acid are preferable. Examples of the alkylsulfonic acid include ethanesulfonic acid and methanesulfonic acid. Examples of the arylsulfonic acid include benzenesulfonic acid and p-toluenesulfonic acid. Examples of the haloalkylsulfonic acid include trifluoromethanesulfonic acid.

An amount of the acid used is preferably 1.0 to 3.0 equivalent (eq.) with respect to 1 equivalent (eq.) of the oxirane derivative (II).

In addition, the acid is preferably added after 4-amino-1,2,4-triazole is dissolved in a solvent of the reaction system.

As the solvent in the reaction of Step 1, a solvent in which the reaction of Step 1 proceeds is appropriately selected, and examples thereof include: alcohols such as methanol, ethanol, 1-propanol, 2-propanol, 1-butanol, and 2-butanol; amides such as N,N-dimethylformamide, N,N-dimethylacetamide, and N-methyl-2-pyrrolidone; ketones such as acetone, methyl ethyl ketone, and cyclohexanone; ethers such as tetrahydrofuran, dimethoxyethane, and dioxane; and nitriles such as acetonitrile and propionitrile. Of these, alcohols and amides are preferable.

The reaction of Step 1 can be performed, for example, at room temperature under stirring or in an oil bath while heating and stirring are performed. As the reaction temperature at this time, for example, the internal temperature is 40 to 120°C.

### Step 2

In Step 2, the amine compound (Ia) obtained in Step 1 is deaminated to form the azole derivative (I).

Step 2 may be performed by using the reaction solution after completion of Step 1as is, that is, Step 2 may be performed in one pot. This eliminates extraction of the amine compound (Ia) and can improve the yield and working efficiency.

The deamination reaction in Step 2 can be progressed by performing a reaction according to a known reaction mechanism for eliminating an amino group bonded to a nitrogen atom. An example of the deamination reaction is a reaction in which an alkali metal nitrite and an acid are added to a reaction solution containing the amine compound (Ia) to eliminate an amino group. Here, the reaction solution containing the amine compound (Ia) may be the reaction solution obtained in Step 1, or may be a solution obtained by extracting the amine compound (Ia) from the reaction solution obtained in Step 1 and dissolving it in another solvent.

Examples of the alkali metal nitrite include sodium nitrite and potassium nitrite, and of these, sodium nitrite is preferable. An amount of the alkali metal nitrite used is preferably 1.0 to 5.0 equivalent (eq.) with respect to 1 equivalent (eq.) of the amine compound (Ia) from the perspective of adequately performing the reaction of Step 2. When the obtained amine compound (Ia) is not quantified, the amount of the alkali metal nitrite may be 1.0 to 5.0 equivalent (eq.) with respect to 1 equivalent (eq.) of the oxirane derivative (II) used in Step 1.

Examples of the acid used together with sodium nitrite include inorganic acids such as hydrochloric acid, sulfuric acid, and nitric acid. An amount of the acid used is preferably 0.1 to 10.0 equivalent (eq.).

Examples of the solvent in the reaction of Step 2 include the solvents listed in the description of Step 1. When the reaction of Step 2 is performed using the reaction solution of Step 1 as is, the solvent contained in the reaction solution is used as is, and the same or a different solvent may be further added.

The reaction of Step 2 may be performed, for example, at a temperature ranging from -10 to 60°C.

In the production method according to the present embodiment, the generation of a 1,3,4-triazole form (the azole derivative (I) is a 1,2,4-triazole form), which is a by-product, can be reduced by synthesizing the azole derivative (I) through Step 1 and Step 2. Thus, the azole derivative (I) with less amount of the by-product and higher purity can be obtained as compared with known production methods. Typically, a ratio of the 1,2,4-triazole form in the reaction product after completion of Step 2 may be 100%. Here, the ratio of the 1,2,4-triazole form in the reaction product is a ratio of the 1,2,4-triazole form when the total amount of the 1,2,4-triazole form and the 1,3,4-triazole form is designated 100%. Hereinafter, the ratio is also referred to as "1,2,4-selectivity".

### Crystallization

The method for producing the azole derivative (I) according to the present embodiment may include purifying the azole derivative (I) by crystallization after Step 2. As described above, in the reaction product obtained through Step 1 and Step 2 of the production method according to the present embodiment, the amount of the 1,3,4-triazole form as a by-product is very small even in a crude product, and the purity of the 1 ,2,4-triazole form is very high. Thus, when crystallization of the azole derivative (I) is performed after Step 2 in the production method of the present embodiment, the target compound can be obtained with high purity without performing hot filtration before the crystallization. Therefore, from the perspective of reducing the work load, in the present embodiment, it is preferable to perform crystallization of the azole derivative without performing hot filtration.

### Summary

The production method according to the present embodiment is a method for producing an azole derivative represented by general formula (I): 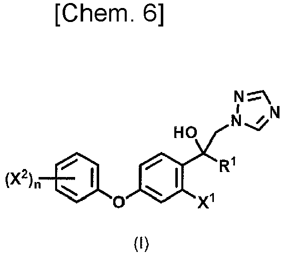
where in general formula (I), R¹ is a C₁-C₆-alkyl group or CO₂R², where R² is a C₁-C₆-alkyl group;
X¹ and X² are each independently a halogen group, a C₁-C₄-haloalkyl group, or a C₁-C₄-haloalkoxy group; and
n is 1, 2, or 3,
the method including:
reacting an oxirane derivative represented by general formula (II) with 4-amino-1,2,4-triazole under an acidic condition to obtain a compound represented by general formula (Ia), 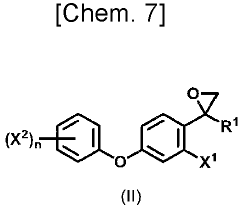 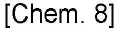 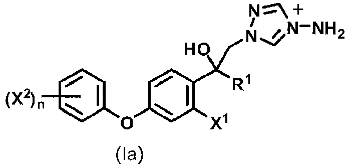
where in general formulae (II) and (Ia), R¹, X¹, X², and n are respectively the same as R¹, X¹, X², and n in general formula (I); and deaminating the obtained compound represented by general formula (Ia) to form an azole derivative represented by general formula (I).

Further, in the production method according to the present embodiment, the acidic condition is preferably formed by containing an alkylsulfonic acid or an arylsulfonic acid.

Further, in the production method according to the present embodiment, a reaction of the deamination is preferably performed by adding an alkali metal nitrite and an acid to a reaction solution containing the compound represented by general formula (Ia).

Further, the production method according to the present embodiment preferably further includes crystallizing the azole derivative represented by general formula (I) without performing hot filtration.

Embodiments of the present invention will be described in further detail hereinafter using examples. The present invention is not limited to the examples below, and it goes without saying that various aspects are possible with regard to the details thereof. Furthermore, the present invention is not limited to the embodiments described above, and various modifications are possible within the scope indicated in the claims. Embodiments obtained by appropriately combining the technical means disclosed by the embodiments are also included in the technical scope of the present invention. In addition, all of the documents described in the present specification are herein incorporated by reference.

### EXAMPLES

### Example 1: Synthesis 1 of methyl 2-(2-chloro-4-(4-chlorophenoxy)phenyl)-2-hydroxy-3-(1H-1,2,4-triazol-1-yl)propionate

To a flask, 9.53 g of a solution of crude methyl 2-(2-chloro-4-(4-chlorophenoxy)phenyl)oxirane-2-carboxylate (GC purity 20.3 wt.%) in dichloroethane was added, and dichloroethane was distilled off. Thereafter, 2.8 mL of 2-butanol, 0.72 g of 4-amino-1,2,4-triazole, and 0.41 mL of methanesulfonic acid were added, and the resulting mixture was heated and stirred in an oil bath at 120°C. After 7 hours from the start of the reaction, the mixture was cooled to room temperature, and then 2.8 mL of N,N-dimethylformamide was added. After cooling with an ice bath, 1.7 mL of concentrated hydrochloric acid and 2.1 mL of a 24 wt.% aqueous sodium nitrite solution was added dropwise. After 20 minutes from the completion of the dropwise addition, ethyl acetate and a saturated aqueous sodium bicarbonate solution were added to terminate the reaction, and the mixture was extracted three times with ethyl acetate. The extract was washed once with saturated brine solution. After drying over anhydrous sodium sulfate, the solvent was distilled off, and thus 4.05 g of an orange liquid crude product was obtained. NMR quantitative yield: 88%. 1,2,4-Selectivity: 100%.

### Example 2: Synthesis 2 of methyl 2-(2-chloro-4-(4-chlorophenoxy)phenyl)-2-hydroxy-3-(1H-1,2,4-triazol-1-yl)propionate

To a flask, 481.12 g of a solution of crude methyl 2-(2-chloro-4-(4-chlorophenoxy)phenyl)oxirane-2-carboxylate (GC purity 14.1 wt.%) in dichloroethane was added, and dichloroethane was distilled off. Thereafter, 100 mL of 2-propanol, 25.22 g of 4-amino-1,2,4-triazole, and 21.17 g of methanesulfonic acid were added, and the resulting mixture was heated under reflux and stirred in an oil bath. After 20 hours from the start of the reaction, the mixture was cooled to room temperature. Then, 75.00 g of concentrated hydrochloric acid and an aqueous solution containing 24.84 g of sodium nitrite and 51.25 g of water were added dropwise. After 30 minutes from the completion of the dropwise addition, 50.00 g of water and 86.69 g of toluene were added to separate the reaction mixture, and the obtained material was re-extracted once with 86.69 g of toluene. Organic phases were combined, washed once with 100.00 g of a 10.6% aqueous sodium carbonate solution, then washed once with 110.92 g of 9% brine solution, and 314.07 g of an orange liquid crude product was obtained. HPLC quantitative yield: 84%. 1,2,4-Selectivity: 100%.

After distilling off the solvent in the crude product, 343.61 g of toluene was added, and the temperature was raised to 100°C in an oil bath. After the internal temperature reached 100°C, heating and stirring were performed for 10 minutes. Then, cooling was performed to 92°C at a rate of 15°C/h. After the temperature reached 92°C, 0.69 g of seed crystals of the title compound were added, and then the resulting material was cooled to 82.5°C at a rate of 15°C/h. After the material reached 82.5°C, stirring was continued for 30 minutes at the same temperature. Thereafter, cooling was performed at a rate of 6°C/h to 75°C, at a rate of 10°C/h from 75°C to 55°C, and at a rate of 30°C/h from 55°C to 5°C, and stirring was continued for 2 hours after the temperature reached 5°C. The crude liquid was vacuum-filtered and washed with 137.42 g of cold toluene. The filtered material was dried under reduced pressure using a vacuum specimen dryer, and thus 60.11 g of a target white solid was obtained. Recovery ratio: 86%. HPLC quantitative purity: 98.4%.

To a flask, 30.06 g of the white solid and 60.00 g of toluene were added, the temperature was raised to 100°C in an oil bath, and after the internal temperature reached 100°C, heating and stirring were performed for 10 minutes. Then, cooling was performed to 92°C at a rate of 15°C/h. After the temperature reached 92°C, 0.36 g of seed crystals of the title compound were added, and stirring was continued for 30 minutes at the same temperature. Thereafter, cooling was performed at a rate of 6°C/h to 75°C, at a rate of 10°C/h from 75°C to 55°C, and at a rate of 30°C/h from 55°C to 5°C, and stirring was continued for 2 hours after the temperature reached 5°C. The crude liquid was vacuum-filtered and washed with 59.2 g of cold toluene. The filtered material was dried under reduced pressure using a vacuum specimen dryer, and thus 28.97 g of a target white solid was obtained. Recovery ratio: 98%. HPLC quantitative purity: 100%.

### Example 3: Synthesis 3 of methyl 2-(2-chloro-4-(4-chlorophenoxy)phenyl)-2-hydroxy-3-(1H-1,2,4-triazol-1-yl)propionate

To a flask, 463.03 g of a solution of crude methyl 2-(2-chloro-4-(4-chlorophenoxy)phenyl)oxirane-2-carboxylate (GC purity 14.7 wt.%) in dichloroethane was added, and dichloroethane was distilled off. Thereafter, 100 mL of 1-propanol, 25.22 g of 4-amino-1,2,4-triazole, and 19.24 g of methanesulfonic acid were added, and the resulting mixture was heated under reflux and stirred in an oil bath. After 17 hours from the start of the reaction, the mixture was cooled to room temperature, and then 44.56 g of concentrated hydrochloric acid was added dropwise. After the internal temperature was cooled to 5°C, an aqueous solution containing 22.08 g of sodium nitrite and 71.48 g of water was added dropwise. After 30 minutes from the completion of the dropwise addition, 8.68 g of toluene was added to separate the reaction mixture, and thus 235.93 g of an orange liquid crude product was obtained. HPLC quantitative yield: 86%. 1,2,4-Selectivity: 100%.

After distilling off the solvent in the crude product, 40.00 g of water was added and then distilled off again. To the resulting material, 173.42 g of toluene was added, and the internal temperature was raised to 80°C, then an aqueous solution containing 10.61 g of sodium carbonate and 100.00 g of water was added dropwise, and the lower phase was separated. The temperature was raised to 100°C in an oil bath, and the resulting material was heated and stirred for 10 minutes after the internal temperature reached 100°C. Then, cooling was performed to 80°C at a rate of 10°C/h. After the temperature reached 80°C, 0.82 g of seed crystals of the title compound were added, and stirring was continued for 30 minutes at the same temperature. Thereafter, cooling was performed at a rate of 10°C/h to 50°C, then at a rate of 20°C/h from 50°C to 5°C, and stirring was continued for 2 hours after the temperature reached 5°C. The crude liquid was vacuum-filtered and washed with 50.00 g, 50.00 g of cold water and 43.34 g, 43.34 g of cold toluene. The filtered material was dried under reduced pressure using a vacuum specimen dryer, and thus 66.65 g of a target white solid was obtained. Recovery ratio: 97%. HPLC quantitative purity: 99.1%.

### Example 4: Synthesis 4 of methyl 2-(2-chloro-4-(4-chlorophenoxy)phenyl)-2-hydroxy-3-(1H-1,2,4-triazol-1-yl)propionate

To a flask, 2327.87 g of a solution of crude methyl 2-(2-chloro-4-(4-chlorophenoxy)phenyl)oxirane-2-carboxylate (GC purity 14.6 wt.%) in dichloroethane was added, and dichloroethane was distilled off. Thereafter, 500 mL of 1-propanol, 401.51 g of 4-amino-1,2,4-triazole, and 96.11 g of methanesulfonic acid were added, and the resulting mixture was heated under reflux and stirred in an oil bath. After 18 hours from the start of the reaction, the mixture was cooled to room temperature, and 1058.48 g of an orange liquid crude product was obtained.

To a flask, 105.85 g of the crude product and 50.01 g of water were added, and the solvent was distilled off. Thereafter, 22.28 g of concentrated hydrochloric acid and 50.00 g of water were added dropwise at an internal temperature of 30°C, and an aqueous solution containing 11.05 g of sodium nitrite and 35.74 g of water was added dropwise. After 30 minutes from the completion of the dropwise addition, 86.69 g of toluene was added, the internal temperature was raised to 60°C, and then the aqueous phase was separated. The internal temperature was raised to 80°C, an aqueous solution containing 5.30 g of sodium carbonate and 25.03 g of water was added dropwise, and then the lower phase was separated. The temperature was raised to 100°C in an oil bath, and the resulting material was heated and stirred for 10 minutes after the internal temperature reached 100°C. Then, cooling was performed to 80°C at a rate of 10°C/h. After the temperature reached 80°C, 0.41 g of seed crystals of the title compound were added, and stirring was continued for 30 minutes at the same temperature. Thereafter, cooling was performed at a rate of 10°C/h to 50°C, then at a rate of 20°C/h from 50°C to 5°C, and stirring was continued for 2 hours after the temperature reached 5°C. The crude liquid was vacuum-filtered and washed with 25.00 g, 25.00 g of cold water and 21.67 g, 21.67 g of cold toluene. The filtered material was dried under reduced pressure using a vacuum specimen dryer, and thus 33.56 g of a target white solid was obtained. HPLC quantitative yield: 81%. 1,2,4-Selectivity: 100%. HPLC quantitative purity: 99.1%.

### Comparative Example 1: Synthesis 5 of methyl 2-(2-chloro-4-(4-chlorophenoxy)phenyl)-2-hydroxy-3-(1H-1,2,4-triazol-1-yl)propionate

To a flask, 69.4 mg of crude methyl 2-(2-chloro-4-(4-chlorophenoxy)phenyl)oxirane-2-carboxylate and 0.5 mL of N,N-dimethylacetamide were added and dissolved. Thereafter, 14.8 mg of triazole and 19.7 mg of triazole sodium salt were added, and the resulting mixture was heated to 60°C and stirred. After 2 hours from the start of the reaction, a saturated aqueous ammonium chloride solution was added to terminate the reaction, and the mixture was extracted with ethyl acetate. The extract was washed three times with water and once with saturated brine solution. After drying over anhydrous sodium sulfate, the solvent was distilled off, and 79.9 mg of a crude product of the title compound was obtained. 1,2,4-Selectivity: 91%.

The crude product was purified by column chromatography (silica gel, ethyl acetate), and 70.8 mg of the title compound as a white solid was thus obtained. Yield: 85%.

In this Comparative Example, both triazole and triazole sodium salt were added, but this does not affect the 1,2,4-selectivity.

### INDUSTRIAL APPLICABILITY

The present invention can be used in production of an azole derivative useful as an agricultural or horticultural chemical.

## Claims

1. A method for producing an azole derivative represented by general formula (I): 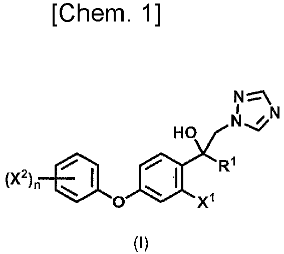 where in general formula (I),
R¹ is a C₁-C₆-alkyl group or CO₂R², where R² is a C₁-C₆-alkyl group;
X¹ and X² are each independently a halogen group, a C₁-C₄-haloalkyl group, or a C₁-C₄-haloalkoxy group; and
n is 1, 2, or 3,
the method comprising:
reacting an oxirane derivative represented by general formula (II) with 4-amino-1,2,4-triazole under an acidic condition to obtain a compound represented by general formula (Ia), 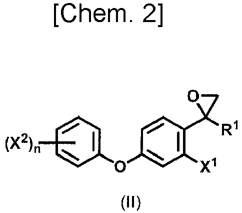 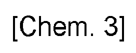 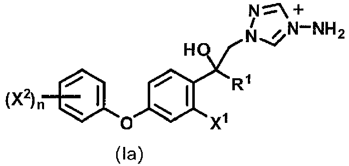
where in general formulae (II) and (Ia),
R¹, X¹, X², and n are respectively the same as R¹, X¹, X², and n in general formula (I); and
deaminating the obtained compound represented by general formula (Ia) to form an azole derivative represented by general formula (I).

2. The method for producing an azole derivative according to claim 1, wherein the acidic condition is formed by containing an alkylsulfonic acid or an arylsulfonic acid.

3. The method for producing an azole derivative according to claim 1 or 2, wherein a reaction of the deamination is performed by adding an alkali metal nitrite and an acid to a reaction solution containing the compound represented by general formula (Ia).

4. The method for producing an azole derivative according to any one of claims 1 to 3, further comprising crystallizing the azole derivative represented by general formula (I) without performing hot filtration.
